# EUROPEAN PATENT APPLICATION

(11) **EP 3 492 018 A1**
(43) Date of publication of application: **05.06.2019**
(21) Application number: 17204726.8
(22) Date of filing: 30.11.2017
(51) Int. Cl.: A61B 8/00, A61B 8/08, A61B 10/02

(54) **DEVICE AND METHOD FOR PROVIDING INFORMATION ABOUT A BIOPSY TAKING PROCESS AND BIOPSY SYSTEM**

(71) Applicant: Koninklijke Philips N.V., 5656 AE Eindhoven (NL)
(72) Inventor: VAN DER ZAAG, Pieter Jan, 5656 AE Eindhoven (NL); STEFFEN, Thomas, 5656 AE Eindhoven (NL)
(74) Representative: de Haan, Poul Erik

(57) **Abstract**

The present invention relates to a device for providing information about a biopsy taking process configured to operate in conjunction with a biopsy taking device (7), the device (1) for providing information about the biopsy taking process comprising: an image acquisition device (6) for acquiring image frames of live anatomy being biopsied, the image acquisition device comprising an electronic processing device (3), a trigger element (2); and a memory (8), preferably being part of the image acquisition device; wherein the trigger element (2) is configured to generate a trigger event to trigger the biopsy taking device (7) for taking a biopsy sample from a biopsy location (9) in said anatomy, and to trigger the image acquisition device (6); and wherein the electronic processing device(3) is configured to: detect the trigger event, upon detection of the trigger event, to select a trigger image frame acquired by the image acquisition device (6) at a predefined point in time after the trigger event, and to store the trigger image frame in the memory (8). The invention provides a facilitated way of determining the biopsy location.

## Description

### FIELD OF THE INVENTION

The present invention relates to a device and a method for providing information about a biopsy taking process and a biopsy system.

### BACKGROUND OF THE INVENTION

To get information about a tumor lesion of a patient, a biopsy sample may be taken from the respective tissue. The biopsy sample may be taken for a traditional pathology workflow in which 2D biopsy slices are examined or in an approach in which the whole 3D (intact) biopsy sample is analyzed, i.e. imaged, intact. Both kind of samples enable a pathological and molecular analysis, e.g. MDx.

From WO 2017/017556 A1 it is known to take a biopsy sample, i.e. to fire a biopsy needle, and to trigger the acquisition of a series of ultrasound image frames covering the biopsy taking process. The trigger simultaneously controls the tip assembly and the imaging of the tip location providing a sequence of images. After the image acquisition, a search algorithm analyzes the series of image frames to find in the frames the image that best captures the fired biopsy needle tip and then to identify its location. For this purpose, the sequence is transferred to a data storage and a processor for providing the location identification.

### SUMMARY OF THE INVENTION

There may thus be a need to provide a facilitated way of providing information about a biopsy taking process.

The object of the present invention is solved by the subject-matter of the independent claims; further embodiments are incorporated in the dependent claims. It should be noted that the following described aspects of the invention apply also for the biopsy system, the method, the computer program element, and the computer readable medium.

According to the present invention, a device for providing information about a biopsy taking process configured to operate in conjunction with a biopsy taking device is provided, the device for providing information about the biopsy taking process comprising: an image acquisition device for acquiring image frames of live anatomy being biopsied, the image acquisition device comprising an electronic processing device, a trigger element; and a memory, preferably being part of the image acquisition device; wherein the trigger element is configured to generate a trigger event to trigger the biopsy taking device for taking a biopsy sample from a biopsy location in said anatomy, and to trigger the image acquisition device; and wherein the electronic processing device is configured to: detect the trigger event, upon detection of the trigger event, to select a trigger image frame acquired by the image acquisition device at a predefined point in time after the trigger event, and to store the trigger image frame in the memory.

It has been found that there is a specific period within which any trigger image frame may be acquired which provides good quality for determining the biopsy location. The trigger element may for example trigger the image acquisition device and the biopsy taking device at the same time, i.e. simultaneously, forming a trigger event. The triggering is performed such that the image acquisition device may acquire just a single trigger image frame, wherein acquiring a single trigger image frame may for example mean acquiring a single trigger image frame with a camera or video, selecting a single image frame from a running video, or putting a marker on a single image frame from a running video. The electronic processing device detects the trigger event and then selects a trigger image frame from the images being acquired by the image acquisition device at a predefined point of time after the trigger event.

That point of time lies within the period of the biopsy taking process in which the biopsy taking device holds its position at the biopsy location wherein the biopsy taking device comprises an outer hollow needle having a sharp tip which may be translated into a patient's body. An inner hollow needle or trocar is arranged in a gliding manner in the outer hollow needle, the inner hollow needle or trocar having a sharp tip. The inner hollow needle or trocar may be fired out of the outer hollow needle and afterwards may be retracted into the outer hollow needle. The firing of the inner hollow needle or trocar results in an extraction of a biopsy sample. Thus, only an inner hollow needle or trocar of the biopsy taking device moves for extracting the biopsy sample.

The single image frame showing the position of the tip of the outer hollow needle of the biopsy taking device when the inner hollow needle or trocar is fired is stored in the memory and thus covers the position at which the biopsy taking device takes a biopsy sample, i.e. provides information about the biopsy taking process.

Thus, it has been found that it is not necessary to provide an image showing the deployed inner hollow needle or trocar of the biopsy taking device. It is only necessary to provide an image which shows the position of tip of the outer hollow needle of the biopsy taking device around the moment, preferably just before or at the moment, of the deployment of the biopsy taking device which may for example mean the firing of the inner hollow needle's tip of the biopsy taking device, since the biopsy taking device does not change its position during the taking process of the biopsy sample. The point in time after the trigger event is chosen such that the position of the biopsy taking device is in the position in which the biopsy has been, is, or will be taken. Therefore, the point in time is such that it does not matter whether, in case of standard deployment with a deployment speed between 10 to 25 mm/s, the biopsy taking process has just been started or, in case of a high-speed deployment with up to 30 m/s, has already been finished. If it is assumed that for example in the standard deployment case, the inner hollow needle or trocar of a biopsy taking device has a length between 10 to 20 mm, the full deployment of the inner hollow needle or trocar will need about 0.5 to 2 seconds, wherein with high-speed deployment the inner hollow needle or trocar will need between 0.3 and 0.9 ms. The biopsy location may be determined considering the well-defined length over which the biopsy is taken, i.e. the well determined length of the biopsy needle deployment. Thus, it is sufficient to locate the tip or end of the outer hollow needle to obtain information about the biopsy location, i.e. to derive the location of the biopsy actually extracted from the tip location.

The present invention therefore avoids taking a series of image frames and thus avoids analysing the series of image frames or searching for an image frame covering the moment of the biopsy sample acquisition in that series of image frames, i.e. showing the deployed inner hollow needle. This simplifies the image acquisition which may result in an acceleration of the image acquisition and image frame determination. Furthermore, this may reduce the required hardware, i.e. the required resources for providing information about the biopsy location are reduced, since less memory is used and no algorithm searches a series of image frames for providing information about the biopsy taking procedure. This may result in a weight and cost reduction.

The image acquisition device may be a distributed image acquisition device, e.g. acquired trigger image frames may be stored remotely, e.g. in a picture archiving and communication system (PACS), or server.

In an example, the image acquisition may be stopped after taking the at least one image frame.

Furthermore, in another example, the electronic processing device may also be triggered by the trigger element. Then, the electronic processing device will only perform a selection if the biopsy taking process is started.

In an example, the electronic process device may be configured to provide the at least one image to display immediately after the image acquisition of the image. The term immediately may for example mean within a predetermined period or without performing an algorithm which determines whether the biopsy taking device is in the image.

In an example, the image acquisition device is configured to acquire image frames with a predefined frequency which may e.g. be 50 Hz, i.e. every 20 ms. In that example, the triggering by the trigger element results in selecting the point in time of the next image frame by the electronic processing device to be taken by the image acquisition device as the at least one image frame. The at least one image frame can be referred to as the first image frame.

In another example, the point in time of the at least one image frame is the only point in time being selected by the electronic processing device for taking an image frame during the biopsy taking process.

According to an example, the predefined point in time is defined within a period of 5 s, preferably within 2.5 s, further preferably within 1 s, more preferably at 500 ms, or most preferably at 20 ms, after the trigger event. The point in time may for example be correlated to a sampling frequency of an image acquisition device, e.g. the point in time may be set to a sampling moment. Furthermore, the predefined point in time may be such that the inner hollow needle will be deployed in any case, i.e. the predefined point in time after the trigger event is defined to be after a point in time in which the inner hollow needle is fully deployed.

According to another example, said period is predefined and stored in memory, and the electronic processing device is configured to select the predefined point in time within said predefined period, randomly. In further preferred example, said period is predefined and stored in memory, and the electronic processing device is configured to select a specific point in time within the predefined period.

According to another example, no such period is stored, or at least it is not used here. The specific point of time is stored in memory (i.e. a fixed value of it), and the electronic processing device is configured to read from the memory the fixed value of the predefined point in time.

This further simplifies the provision of information of the biopsy location. As stated above, it is sufficient to locate the tip of the outer hollow needle since the biopsy taking device does not move during the biopsy taking process, any point in time during the biopsy taking process, i.e. when the biopsy taking device is in the position in which the deployment occurs, may be selected for acquiring an image frame of the biopsy location and the biopsy taking device. Besides, as also said the distance of the extremity of the inner hollow needle between the un-deployed and deployed state is known by construction, and so can be the precise location of the biopsy extraction if needed.

According to an example, the device further comprises: a delay module; wherein the delay module is configured to trigger the image acquisition device after a further predefined period after the trigger event to select a further trigger image frame; wherein the further predefined period preferably ranges from 1 s to 3 s, further preferably from 1.5 s to 2.5 s, or is further preferably 2 s.

The further trigger image may show the biopsy taking device in a deployed state. In the deployed state, an inner hollow needle or trocar of the biopsy taking device may be deployed, i.e. the inner hollow needle's tip may the shown in the further image frame in a fully extracted position.

The acquisition of a further trigger image may be applied without further consideration in biopsy taking system which do not automatically retract the inner hollow needle. If the biopsy taking system automatically retracts the inner hollow needle, the delay must be precisely adapted to the period of the deployment of the inner hollow needle.

In an example, the electronic processing device is configured to provide the further trigger image frame of the biopsy location to display.

The further trigger image frame can also be referred to as second image frame. With the further image frame, the biopsy location may be imaged with the fully for nearly fully deployed biopsy taking device. The biopsy location can therefore be compared between two points in time during or around the biopsy taking process. The delay module may be arranged on or integrated into the trigger element.

According to an example, the trigger element is configured to repeatedly generate trigger events, wherein at each trigger event, the biopsy taking device performs a biopsy taking process at a different biopsy location.

This allows multiple biopsies to be taken at different locations in one session without waiting for a search algorithm to provide an image of the biopsy location. The biopsy taking device may be moved from one biopsy location to another biopsy location between two trigger events. The biopsy taking device may also be moved outside of the patient and inside again between the trigger events. A process in which multiple biopsies are taken may therefore be accelerated since each triggering triggers the taking of a biopsy sample and the acquisition of a trigger image frame of the biopsy location.

The delay module may save the timing of the repeated trigger pulses and provide a trigger for a further trigger image frame for each repeated trigger event.

According to the present invention, also a biopsy system is provided. The biopsy system comprises: a biopsy taking device for taking a biopsy sample of a region of interest; and a device for providing information about a biopsy taking process according to one of the preceding claims; wherein the trigger element of the device for providing information about a biopsy taking process is connected to the biopsy taking device and the image acquisition device.

In an example, the system further comprises: a display element; wherein the display element is configured to receive an image from the controller and to display the received image.

According to an example, the image acquisition device is an ultrasound device. In certain exemplary cases, also other imaging systems could be considered such as an X-ray system or an MRI system.

According to the present invention, also a method for determining a biopsy location is provided. The method comprises the following steps of: a) acquiring image frames of live anatomy being biopsied by means of an image acquisition device comprising an electronic processing device; b) generating a trigger event by triggering a biopsy taking device for taking a biopsy sample from a biopsy location in said anatomy and the image acquisition device with a trigger element; and c) selecting a trigger frame acquired at a predefined point in time after the trigger event with an electronic processing device d) storing said trigger image frame in a memory.

In an example, the method may comprise the further step of providing the trigger image frame of the biopsy location to display immediately after the image acquisition of the image to determine the biopsy location.

According to an example, step c) is performed within a period of 5 s, preferably within 2.5 s, further preferably within 1 s, more preferably at 500 ms, or most preferably at 20 ms, after the trigger event.

According to a further example, the trigger event simultaneously triggers the biopsy taking device and the image acquisition device.

According to an example, the method further comprises the step of: e) triggering the image acquisition device after a further predefined period after the trigger event to take select a further trigger image frame; wherein the further predefined period is ranges from between 1 s and to 3 s, preferably between from 1.5 s and to 2.5 s, or is further preferably 2.s

In a further example, the method may comprise the step of: providing the further trigger image frame of the biopsy location by the electronic processing device to display.

According to an example, the method further comprises the step of: f) repeating at least steps b) to d) at different biopsy locations.

According to the present invention, also a computer program element for controlling an apparatus according to the above description, which, when being executed by a processing unit, is adapted to perform the method steps according to the above description is provided.

According to the present invention, also a computer readable medium having stored the program element according to the above description is provided.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic drawing of the system comprising the device for determining a biopsy location.
Fig. 2 shows a schematic timeline of the triggering during the image sampling.
Fig. 3 shows a schematic flow chart of the method.

### DETAILED DESCRIPTION OF EMBODIMENTS

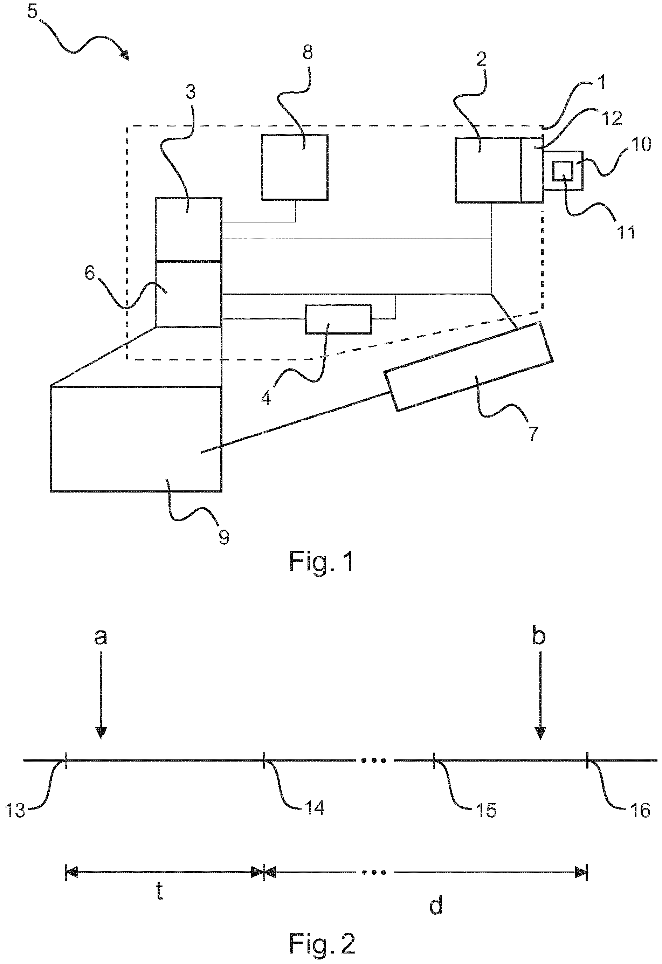

Fig. 1 shows a biopsy system 5 comprising a device 1 for providing information about a biopsy taking process at a biopsy location 9, and a biopsy taking device 7. Furthermore, the system 5 comprises a display (not shown) for displaying an image frame being acquired by the image acquisition device 6.

The biopsy taking device 7 is introduced into a patient's body to a biopsy location 9. When the biopsy taking device 7 is triggered, a biopsy sample of the biopsy location 9 will be extracted from a region of interest, i.e. the biopsy taking process is performed. The biopsy taking device 7 may for example be a biopsy gun having an inner hollow needle or a trocar with a needle tip being arranged in an outer hollow needle. The triggering of the biopsy taking device 7 will deploy the inner hollow needle's tip such that the inner hollow needle's tip will enter the biopsy location 9 and extract a biopsy sample.

Depending on the deployment velocity and the travelling distance of the inner hollow needle's tip of the biopsy taking device, the extraction will happen when the inner hollow needle's tip is fully deployed at the biopsy location inside a patient. For a deployment velocity of 10 mm/s and a travelling distance of 10 mm, the extraction of the biopsy sample will take about 2 seconds, i.e. 1 s until the inner hollow needle's tip travels the full distance out of the biopsy taking device 7 and 1s until the inner hollow needle's tip is fully retracted into the biopsy taking device 7.

The device 1 comprises an image acquisition device 6 comprising an electronic processing device 3, a trigger element 2, an, a memory 8, and a delay module 4.

The biopsy location 9 comprising the region of interest is covered by an acquisition area of the image acquisition device 6. The image acquisition device 6 may be an ultrasound device having a frame rate of 50 Hz.

The trigger element 2 can be connected to the image acquisition device 6 and to the biopsy taking device 7. The connection is such that a trigger signal may be provided by the trigger element 2 to the image acquisition device 6 and to the biopsy taking device 7, generating a trigger event.

In one embodiment, the trigger may simultaneously trigger the biopsy taking device 7 and the image acquisition device 6. In another embodiment, the trigger element 2 may provide two trigger signals, one for the biopsy taking device 7 and one for the image acquisition device 7, wherein the two trigger signals may have a predefined delay period in between. The predefined delay period may be for example between 20 ms and 100 ms.

The trigger signal may be received by the electronic processing device 3. In the embodiment shown in Fig. 2, the trigger element is arranged in the device 1. In an alternative embodiment (not shown), the trigger element 2 may be arranged on the gun of the biopsy taking device 7, e.g. as a trigger button (not shown). In alternative embodiment, the trigger element 2 may be arranged somewhere else, for example at a convenient place from the point of view of the clinician performing the interventional biopsy taking procedure.

The triggering of the biopsy taking device 7 starts the biopsy taking process. Furthermore, the image acquisition device 6 is triggered to acquire a trigger image frame of the biopsy location 9 and of a portion of the biopsy taking device 7 being arranged at the biopsy location 9 selected by the electronic processing device 3 at a point in time after the trigger event. This trigger image frame may be referred to be the first image frame.

The point in time after the trigger event is such that is lies within the period of the biopsy taking process which may be 5 s, preferably within 2.5 s, further preferably within 1 s, more preferably at 500 ms, or most preferably at 20 ms. The predetermined period may be shorter depending on the deployment velocity of the biopsy taking device 7 and the travelling distance of the inner hollow needle or trocar of the biopsy taking device 7. Furthermore, this predetermined period may be longer if it is known how long the biopsy taking device 7 will stay in position after the end of the biopsy taking process.

In a first embodiment, the electronic processing device 3 may select the trigger frame at a specific point in time or within a specific predefined period after the trigger event which is shorter than or equal to the period of the biopsy taking process. The specific period may be correlated to a sampling time of the image acquisition device or may be a number of an image frame at a sampling time point after the trigger event, e.g. image frame no. 1 after the trigger event, i.e. the next image frame, or the image frame no. 10 after the trigger event, etc.

In a second embodiment, the electronic processing device 3 may randomly select a trigger image frame for acquiring an image frame with the image acquisition device 6, wherein the trigger image frame lies at a point in time within the predefined period after the trigger event.

The trigger image frame may be stored in the memory 8 for further processing and/or use by an operator of the biopsy system 5.

In one embodiment, the memory 8 may be integrated into the image acquisition system 6.

In an alternative embodiment, the memory 8 may be a component of an external and/or remote storage, e.g. a PACS system or a server.

Furthermore, the trigger element 2 may provide the trigger signal to the delay module 4. The delay module 4 may memorize the point in time at which it receives the trigger signal. After a certain period, the delay module 4 provides a further trigger signal to the image acquisition device 6, i.e. generates a further trigger event for selecting a further trigger frame. The further trigger signal triggers the image acquisition device 6 to take a further trigger image frame of the biopsy location 9. The further image frame may be referred to be the second image frame.

The delay being provided by the delay module 4 may be two seconds. This means, that the further image frame will provide information about the biopsy location 9 two seconds after the trigger event. In a standard deployment case, after those two seconds, an inner hollow needle or a trocar of the biopsy taking device 7 has reached the biopsy location 9 such that the further trigger image frame shows the biopsy location 9 during the biopsy taking process.

Hereby, the image acquisition device 6 may provide the first and the second image frame to the electronic processing device. The electronic processing device may send the trigger image frames to the memory 8 and/or to the display device of the system 5. The display device may display the trigger image frames to a user.

The trigger element 2 may be configured to repeatedly trigger the biopsy taking device 7 and the image acquisition device 6 during a single biopsy taking session, wherein the biopsy taking device 7 performs several biopsy taking processes at different biopsy locations 9. This means that multiple biopsies may be taken from the patient in a single biopsy session. For each of the multiple biopsies, the image acquisition device 6 will acquire image frames and the electronic processing device may select a trigger image frame at a point in time after the trigger event, the trigger image frame showing the biopsy location 9 while the biopsy taking device 7 takes a biopsy sample.

Fig. 2 shows a timeline having sampling times 13, 14, 15, 16 of the image acquisition device 6 to further describe the image acquisition process. This exemplary embodiment comprises an ultrasound device having a frame rate, i.e. sampling frequency, of 50 Hz, the period t between two sampling times may be 20 ms. At a point in time a, a trigger signal may be provided by the trigger element 2, i.e. the trigger event is generated. The trigger signal will be provided between sampling time 13 and sampling time 14. In this embodiment, the electronic processing device 3 is configured to select the next image frame which can be taken by the image acquisition device 6 as trigger image frame. Upon the receiving of the trigger signal, the image acquisition device 6 may therefore take the image frame at the sampling time 14, i.e. image frame no. 1 after the triggering, and provide that image frame as image of the biopsy location showing the biopsy taking device 7. Thus, the electronic processing device 3 will take the first image frame after the trigger signal is provided by the trigger element 2 as trigger image frame. The predefined point in time is then at the sampling time after the trigger event.

The further image frame may be acquired by the image acquisition device 6 after a delay time d, when the delay module 4 provides a further, i.e. a second, trigger signal to the image acquisition device 6 at the point in time b. When using high-speed deployment, at this point in time, the biopsy taking process has terminated and the further trigger image frame being triggered by the second trigger signal will provide information about the biopsy location after the biopsy taking procedure. When using a standard deployment, the inner hollow needle or trocar of the biopsy taking device 7 will be fully deployed and the further trigger image may provide further information about the biopsy taking process.

In another alternative embodiment, the image acquisition device 6 may be a camera. The electronic processing device 3 will then select a point in time at which the camera will take an image of the biopsy location.

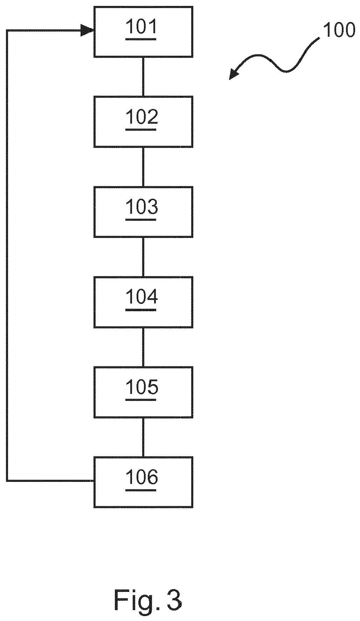

A method 100 for determining a biopsy location is shown in Fig. 3 as a schematic flow chart diagram. The method 100 may be performed with a biopsy system 5 comprising a device 1 for providing information about a biopsy taking process at a biopsy location 9, and a biopsy taking device 7. Furthermore, the system 5 comprises a display (not shown) for displaying an image frame being acquired by the image acquisition device 6.

The biopsy taking device 7 is introduced into a patient's body to a biopsy location 9. When the biopsy taking device 7 is triggered, a biopsy sample of the biopsy location 9 extracted from a region of interest, i.e. the biopsy taking process is performed. The biopsy taking device 7 may for example be a biopsy gun having an inner hollow needle or a trocar with a needle tip being arranged in an outer hollow needle. The triggering of the biopsy taking device 7 will deploy the inner hollow needle's tip such that the inner hollow needle's tip will enter the biopsy location 9 and extract a biopsy sample.

Depending on the deployment velocity and the travelling distance of the inner hollow needle's tip of the biopsy taking device, the extraction will happen when the inner hollow needle's tip of the biopsy taking device is fully deployed at the biopsy location inside a patient. For a deployment velocity of 10 mm/s and a travelling distance of 10 mm, the extraction of the biopsy sample will take about 2 seconds, i.e. 1 s until the inner hollow needle's tip travels the full distance out of the biopsy taking device 7 and Is until the inner hollow needle's tip is fully retracted into the biopsy taking device 7.

The device 1 comprises an image acquisition device 6 comprising an electronic processing device 3, a trigger element 2, a memory 8, and a delay module 4.

The biopsy location 9 comprising the region of interest is covered by an acquisition area of the image acquisition device 6. The image acquisition device 6 maybe an ultrasound device having a frame rate of 50 Hz. The image acquisition device 6 acquires 101 image frames of human anatomy being biopsied according to step a).

The trigger element 2 can be connected to the image acquisition device 6 and to the biopsy taking device 7. The connection is such that a trigger signal may be provided by the trigger element 2 to the image acquisition device 6 and to the biopsy taking device 7, generating a trigger event.

According to step b) of the method 100, the biopsy taking device the image acquisition device and triggered 102.

In one embodiment, the trigger may simultaneously trigger the biopsy taking device 7 and the image acquisition device 6. In another embodiment, the trigger element 2 may provide two trigger signals, one for the biopsy taking device 7 and one for the image acquisition device 7, wherein the two trigger signals may have a predefined delay period in between.

The trigger signal may be received by the electronic processing device 3. In the embodiment shown in Fig. 2, the trigger element is arranged in the device 1. In an alternative embodiment (not shown), the trigger element 2 may be arranged on the gun of the biopsy taking device 7, e.g. as a trigger button (not shown).

The triggering of the biopsy taking device 7 starts the biopsy taking process. Furthermore, the image acquisition device 6 is triggered to acquire a trigger image frame of the biopsy location 9 and of a portion of the biopsy taking device 7 being arranged at the biopsy location 9 selected by the electronic processing device 3 at a point in time after the trigger event. This trigger image frame may be referred to be the first image frame.

The point in time after the trigger event is such that is lies within the period of the biopsy taking process which may be 5 s, preferably within 2.5 s, further preferably within 1 s, more preferably at 500 ms, or most preferably at 20 ms. The predetermined period may be shorter depending on the deployment velocity of the biopsy taking device 7 and the travelling distance of the inner hollow needle or trocar of the biopsy taking device 7. Furthermore, this predetermined period may be longer if it is known how long the biopsy taking device 7 will stay in position after the end of the biopsy taking process.

In a first embodiment, the electronic processing device 3 may select the trigger frame at a specific point in time or within a specific predefined period after the trigger event which is shorter than or equal to the period of the biopsy taking process. The specific period may be correlated to a sampling time of the image acquisition device or may be a number of an image frame at a sampling time point after the trigger event, e.g. image frame no. 1 after the trigger event, i.e. the next image frame, or the image frame no. 10 after the trigger event, etc.
In a second embodiment, the electronic processing device 3 may randomly select a trigger image frame for acquiring an image frame with the image acquisition device 6, wherein the trigger image frame lies at a point in time within the predefined period after the trigger event. The triggering 102 of the image acquisition device 6 results in an selecting 103 of a trigger image frame of the biopsy location according to step c).

According to a further step, the image acquisition device 6 may provide the trigger image frame to the electronic processing device 3 immediately after the image acquisition. The electronic processing device 3 may prepare the trigger image frame to display on a display device of the system 5. Moreover, the electronic processing device 3 may provide the prepared trigger image frame to the display device. The display device may display the prepared trigger image frame to a user.

The trigger image frame may be stored 104 in the memory 8 for further processing and/or use by an operator of the biopsy system 5 according to step d).

In one embodiment, the memory 8 may be integrated into the image acquisition system 6.

In an alternative embodiment, the memory 8 may be a component of an external and/or remote storage, e.g. a PACS system or a server.

Furthermore, the trigger element 2 may provide the trigger signal to the delay module 4. The delay module 4 memorizes the point in time at which it receives the trigger signal. After a certain period, the delay module 4 provides a further trigger signal to the image acquisition device 6, i.e. generates a further trigger event for selecting a further trigger frame. The further trigger signal triggers the image acquisition device 6 to acquire 105 a further trigger image frame of the biopsy location 9 according to step e). The further image frame may be referred to be the second image frame.

The delay being provided by the delay module 4 may be two seconds. This means, that the further image frame will provide information about the biopsy location 9 after the trigger event. In a standard deployment case, after those two seconds, an inner hollow needle or a trocar of the biopsy taking device 7 has reached the biopsy location 9 such that the further trigger image frame shows the biopsy location 9 during the biopsy taking process.

Hereby, the image acquisition device 6 may provide the first and the second image frame to the electronic processing device 3. The electronic processing device 3 may send the trigger image frames to the memory 8 and/or to a display device of the system 5. The display device may display the trigger image frames to a user.

The trigger element 2 may be configured to repeatedly trigger the biopsy taking device 7 and the image acquisition device 6 during a single biopsy taking session, wherein the biopsy taking device performs several biopsy taking processes at different biopsy locations 9. This means that multiple biopsies may be taken from the patient in a single biopsy session. For each of the multiple biopsies, the image acquisition device 6 will acquire image frames and the electronic processing device 3 may select a trigger image frame at a point in time after the trigger event, the trigger image frame showing the biopsy location 9 while the biopsy taking device 7 takes a biopsy sample. According to step f), this means that the method 100 is repeated 106.

Fig. 2 shows a timeline having sampling times 13, 14, 15, 16 of the image acquisition device 6 to further describe the image acquisition process. This exemplary embodiment comprises an ultrasound device having a frame rate, i.e. sampling frequency, of 50 Hz, the period t between two sampling times may be 20 ms. At a point in time a, a trigger signal may be provided by the trigger element 2, i.e. the trigger event is generated. The trigger signal will be provided between sampling time 13 and sampling time 14. In this embodiment, the electronic processing device 3 is configured to select the next image frame which can be taken by the image acquisition device 6 as trigger image frame. Upon the receiving of the trigger signal, the image acquisition device 6 may therefore take the image frame at the sampling time 14, i.e. image frame no. 1 after the triggering, and provide that image frame as image of the biopsy location showing the biopsy taking device 7. Thus, the electronic processing device 3will take the first image frame after the trigger signal is provided by the trigger element 2 as trigger image frame. The predefined point in time is then at the sampling time after the trigger event.

The further image frame may be acquired by the image acquisition device 6 after a delay time d, when the delay module 4 provides a further, i.e. a second, trigger signal to the image acquisition device 6 at the point in time b. When using high-speed deployment, at this point in time, the biopsy taking process has terminated and the further trigger image frame being triggered by the second trigger signal will provide information about the biopsy location after the biopsy taking procedure. When using a standard deployment, the inner hollow needle or trocar of the biopsy taking device 7 will be fully deployed and the further trigger image may provide further information about the biopsy taking process.

In another alternative embodiment, the image acquisition device 6 may be a camera. The electronic processing device 3 will then select a point in time at which the camera will take an image of the biopsy location.

In another exemplary embodiment of the present invention, a computer program 11 or a computer program element 11 is provided that is characterized by being adapted to execute the method steps of the method 100 according to one of the preceding embodiments, on an appropriate system.

The computer program element 11 might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor 12. The data processor 12 may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element 11 might be able to provide all necessary steps to fulfil the procedure of an exemplary embodiment of the method as described above.

According to a further exemplary embodiment of the present invention, a computer readable medium 10, such as a CD-ROM, is presented wherein the computer readable medium has a computer program element stored on it which computer program element 11 is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It should be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features. For example, a person skilled in the art will easily derive that some of the components described in Fig.1 may be distributed differently in the system. For instance, device 1 may also include the image acquisition device 6, the memory 8 and the display for showing the specific image frame. Actually, the memory 8 and the display could be part of the image acquisition device 6. While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device for providing information about a biopsy taking process configured to operate in conjunction with a biopsy taking device (7), the device (1) for providing information about the biopsy taking process comprising:
- an image acquisition device (6) for acquiring image frames of live anatomy being biopsied, the image acquisition device comprising an electronic processing device (3),
- a trigger element (2); and
- a memory (8), preferably being part of the image acquisition device;
wherein the trigger element (2) is configured to generate a trigger event to trigger the biopsy taking device (7) for taking a biopsy sample from a biopsy location (9) in said anatomy, and to trigger the image acquisition device (6); and
wherein the electronic processing device(3) is configured to:
- detect the trigger event,
- upon detection of the trigger event, to select a trigger image frame acquired by the image acquisition device (6) at a predefined point in time after the trigger event, and
- to store the trigger image frame in the memory (8).

2. Device according to claim 1, wherein the predefined point in time is defined within a period of 5 s, preferably within 2.5 s, further preferably within 1 s, more preferably at 500 ms, or most preferably at 20 ms, after the trigger event.

3. Device according to claim 2, wherein said period is predefined and stored in the memory, and the electronic processing device (3) is configured to randomly select the predefined point in time within said predefined period or is configured to select a specific point in time within the predefined period, or wherein the electronic processing device is configured to read from the memory a fixed value of the predefined point.

4. Device according to one of the preceding claims, wherein the trigger event simultaneously triggers the biopsy taking device (7) and the image acquisition device (6).

5. Device according to one of claims 1 to 4, wherein the device (1) further comprises:
- a delay module (4);
wherein the delay module (4) is configured to trigger the image acquisition device (6) after a further predefined period after the trigger event to select a further trigger image frame;
wherein the further predefined period preferably ranges from 1 s to 3 s, further preferably from 1.5 s to 2.5 s, or is further preferably 2 s.

6. Device according to one of claims 1 to 4, wherein the trigger element (2) is configured to repeatedly generate trigger events, wherein at each trigger event the biopsy taking device (7) performs a biopsy taking process at a different biopsy location 9.

7. A biopsy system (5) comprising:
- a biopsy taking device (7) for taking a biopsy sample of a region of interest; and
- a device (1) for providing information about a biopsy taking process according to one of the preceding claims;
wherein the trigger element (2) of the device (1) for providing information about a biopsy taking process is connected to the biopsy taking device (7) and the image acquisition device (6).

8. System according to claim 7, wherein the image acquisition device (6) is an ultrasound device.

9. A method for providing information about a biopsy taking process, the method (100) comprising the following steps:
a) Acquiring (101) image frames of live anatomy being biopsied by means of an image acquisition device comprising an electronic processing device;
b) Generating (102) a trigger event by triggering a biopsy taking device for taking a biopsy sample from a biopsy location in said anatomy and the image acquisition device with a trigger element; and
c) Selecting (103) a trigger frame acquired at a predefined point in time after the trigger event with an electronic processing device
d) Storing (104) said trigger image frame in a memory.

10. Method according to claim 9, wherein step c) is performed within a period of 5 s, preferably within 2.5 s, further preferably within 1 s, more preferably at 500 ms, or most preferably at 20 ms, after the trigger event.

11. Method according to claim 9 or 10, wherein the trigger event simultaneously triggers the biopsy taking device and the image acquisition device.

12. Method according to one of claims 9 or 11, wherein the method (100) further comprises the step:
e) Triggering (105) the image acquisition device after a further predefined period after the trigger event to select a further trigger image frame;
wherein the further predefined period ranges from 1 s to 3 s, preferably from 1.5 s to 2.5 s, or is further preferably 2 s.

13. Method according to one of claims 9 to 12, wherein the method further comprises the step:
f) Repeating (106) at least steps b) to d) at different biopsy locations.

14. A computer program element for controlling an device according to one of the claims 1 to 6 or the system according to one of the claims 7 and 8, which, when being executed by a processing unit (12), is adapted to perform the method steps of one of the claims 9 to 13.

15. A computer readable medium having stored the program element of claim 14.
